# EUROPEAN PATENT SPECIFICATION

(11) **EP 4 237 024 B1**
(45) Date of publication and mention of the grant of the patent: **07.05.2025**
(21) Application number: 21805467.4
(22) Date of filing: 02.11.2021
(51) Int. Cl.: A61L 27/22

(54) **IMPLANT FOR NERVE REGENERATION**
IMPLANTAT ZUR NERVENREGENERATION
IMPLANT POUR RÉGÉNÉRATION NERVEUSE

(30) Priority: 02.11.2020 EP 20205219
(43) Date of publication of application: 06.09.2023
(73) Proprietor: Medizinische Hochschule Hannover, 30625 Hannover (DE)
(72) Inventor: VOGT, Peter, 30625 Hannover (DE); LIEBSCH, Christina, 30625 Hannover (DE); BUCAN, Vesna, 30625 Hannover (DE); HAVERICH, Axel, 30625 Hannover (DE); STRAUSS, Sarah, 30625 Hannover (DE); APER, Thomas, 30625 Hannover (DE); WILHELMI, Mathias, 30625 Hannover (DE)
(74) Representative: Taruttis, Stefan Georg
(86) International application number: PCT/EP2021/080327
(87) International publication number: WO 2022/090563

(56) References cited:
- WO-A1-2008/140413
- US-A1- 2009 099 580
- US-A1- 2016 074 042

## Description

The present invention relates to an implant suitable for nerve regeneration, also referred to as a neural implant. The implant contains silk fibers arranged longitudinally in a sheath, which provides for mechanical stability suitable for handling the implant, e.g. during surgery, and suitable for connecting the implant to tissue, e.g. connecting its ends to nerve stumps. The implant is especially suitable for bridging defect sites of nerves, especially defect sites within the central nervous system, e.g. for spanning or bridging defects of axons within the spinal cord, or for spanning defects of peripheral nerves, of motor nerves or of sensory nerves. It was found that the implant in a recipient allows the migration and growth of neurites, e.g. of dorsal root ganglia, along the silk fibers of the implant. Defect sites of nerves are e.g., interruptions or gaps within a nerve.

Further, the invention provides a process for producing the implant, which has the advantage of not comprising any steps comprising mammalian cells, e.g. the process is devoid of cultivating cells or integrating cells into the implant, and preferably the process is devoid of any cell growth factors or hormones, e.g. the process preferably does not involve integrating any cell growth factors or hormones into the implant.

In a preferred embodiment, the implant has a sheath that consists of polymerized natural compounds and the process for producing the sheath of the implant only uses natural compounds, e.g. polymerization of only natural compounds.

Further, the invention provides a method for bridging defects of nerves, especially bridging defects of axons within the spinal cord, by implanting the implant of the invention into the site of the defect and arranging the nerve stumps that border the defect site adjacent to the implant, preferably adjacent to the end cross-sections of the implant. Preferably, the nerve stumps that delimit the defect of nerves are connected to the implant, especially connected to the silk fibers arranged at the terminal cross-sections of the implant. Nerve stumps can be connected to the silk fibers of the implant by gluing, e.g. using fibrin glue, and/or by sewing. The defect site of a nerve can be a recent defect, e.g. caused by mechanical interruption of the nerve, e.g. within 2 h up to 2 d prior to inserting the implant into the defect site, or the defect site can be an old mechanical interruption of the nerve, e.g. at least 2 days or more prior to inserting the implant. It was found that especially for defects of nerves like interruptions of the spinal cord, the implant and the method of the invention are suitable also for use in treatment of old defects.

### State of the art

US 2009/099580 A1 for a medical device describes a tubular body which can be composed of silk fibres helically wound around a cylindrical former and then cross-linked with formaldehyde, optionally treated with regenerated silk. In the tube, silk filaments are arranged and the lumen optionally is filled with fibrin glue, hyaluronic acid, hyaluronic acid with polylysine, alginate with or without polylysine and casein.

US 2016/0074042 A1 describes neural implants comprising spider silk fibers within a sheath of spide silk fibres, optionally cross-linked, or within an acellularized venule, or spider silk fibres wound around a carrier of spider silk fibres or of a strip-shaped venule or surgical suture material.

WO 2017/005857 A1 describes a process for producing a tube of high density fibrin that is suitable as an implant and as a matrix for a blood vessel prosthesis.

WO2013/091865 A1 describes a process for producing a tissue construct that contains living mammalian cells by casting cells and fibrinogen in a rotating tubular mould.

WO2008/140416 A1 describes a process for producing a fibrin-based nerve repair conduit by curing a tissue glue forming fibrinogen and serin protease in a mould having a central shaft. Wang et al., The Anatomical Record 301: 1690-1696 (2018) describe fibrin conduits consisting of fibrin obtained by statically pressing two-compound fibrin glue in a silicone mould, and after polymerization using the conduits as implants for repairing nerve defects.

WO2007/028801 A2 describes a neural implant of spider silk that can consist of braided spider silk or of spider silk arranged in an acellularized venule.

US 8 106 014 B2 describes a neural implant of silkworm silk fibers arranged in a biodegradable tube.

US2005/0260706 A1 describes a tissue engineered construct comprising electrospun fibers of silk protein, which preferably is fibroin from dissolved silkworm silk, in a mixture with a polymer, e.g. polyethylene oxide, PEG, collagen, fibronectin, keratin, polyaspartic acid, polylysine, alginate, chitosan, chitin, hyaluronic acid, pectin, polycaprolactone, polylactic acid, polyglycolic acid, polyhydroxy alkanoates, dextrans, and polyanhydrides.

EP2097117 B1 describes an implant suitable as a scaffolding matrix which is produced by moulding and cross-linking spider silk threads.

### Object of the invention

It is an object of the invention to provide an alternative neural implant, preferably a neural implant suitable for bridging. Preferably, the neural implant has a structure that promotes nerve growth, e.g. along the implant. More preferably, the neural implant shall be easy to handle and shall have a mechanical stability that allows connecting it with nerve stumps in a recipient, and the neural implant shall be accessible by a simple production method.

### Description of the invention

The invention achieves the object by the features of the claims, and especially by an implant that is especially suitable for bridging defect sites of nerves, especially defect sites within the central nervous system, e.g. for spanning or bridging defects of axons within the spinal cord, which implant comprises or consists of
a sheath, which preferably about its axis is circumferentially closed between its ends, the sheath consisting of fibrin and
silk fibers arranged in axial direction inside the sheath,
fibrin filling the inner volume of the sheath and between the silk fibers.

The ends of the sheath can form an open cross-section that is enclosed by the sheath, and optionally the inner volume of the sheath and between the silk fibers can be filled with fibrin, e.g. up to the cross-section of the sheath.

The sheath preferably is circumferentially closed, e.g. by the sheath being continuous and having a closed surface. Alternatively, the sheath can be in the form of a strip-shape that is arranged, e.g. wound around the silk fibers, wherein preferably the long edges of the strip-shape overlap. A sheath in the form of a strip-shape can be produced by first producing a circumferentially closed sheath by polymerizing fibrinogen to fibrin in a rotating mould, removing the circumferentially closed sheath from the mould and cutting the circumferentially closed sheath into a strip-shape, e.g. cutting lengthwise or spirally, e.g. after inserting a support, e.g. a Teflon-coated round steel rod, into the circumferentially closed sheath.

The process of the invention for producing the implant comprises or consists of producing a sheath consisting of fibrin, the sheath extending along a longitudinal axis, preferably drying the sheath before or after arranging silk fibers in the sheath to generate a dried sheath, and, before or subsequent to arranging silk fibers in the sheath, re-hydrating the dried sheath to generate a re-hydrated sheath,
arranging silk fibers in the sheath, the fibers extending along the longitudinal axis of the sheath,
and filling the inner volume of the sheath and between the silk fibers with fibrin, e.g. by introducing fibrinogen and, concurrently or subsequently, thrombin into the inner sheath volume.

Preferably, the sheath consists of fibrin having a high density, e.g. obtainable by polymerizing a fibrinogen containing solution in a rotating mould, which preferably about its longitudinal axis is rotationally symmetrical and has a constant diameter and rotates to generate a g-force of at least 200 x g, e.g. at least or up to 900 x g, preferably 300 x g to 500 x g, e.g. 400 x g, the g-force being determined at the inner wall of the rotating mould.

Optionally, olfactory ensheathing cells (OEC) are introduced into the fibrin sheath, e.g. embedded in the fibrin filling the inner volume of the fibrin sheath. Olfactory ensheathing cells (OEC) can e.g. be autologous cells derived from the later recipient of the implant, and OEC can be increased in number by cultivating OEC derived from the later recipient of the implant prior to introducing the OEC into the sheath.

Optionally, the silk fibers can be arranged in the sheath while the sheath has a state of hydration that originates from its production.

Preferably, an insert, e.g. of plastics or steel, is inserted into the fibrin sheath prior to drying, and drying the fibrin sheath with the insert inserted, before or after arranging silk fibers in the fibrin sheath to generate a dried fibrin sheath, and, before or subsequent to arranging silk fibers in the fibrin sheath, re-hydrating the dried fibrin sheath to generate a re-hydrated fibrin sheath, optionally removing the insert from the dried fibrin sheath before or after re-hydrating. The insert that is inserted into the fibrin sheath prior to drying has the advantage of determining the inner diameter of the dried fibrin sheath, and it has been found that an insert of plastics or of steel can easily be withdrawn from the sheath prior to or after re-hydrating the dried fibrin sheath. Further, the insert present in the fibrin sheath during the drying preferably also determines the inner diameter of the fibrin sheath after re-hydrating it, e.g. preferably determines the inner diameter of the re-hydrated fibrin sheath to be the outer diameter of the insert.

The mould preferably has a smooth inner surface, e.g. of steel, preferably of polytetrafluorethylene (available as Teflon) or polyetheretherketone (PEEK), and the mould has drainage bore holes for draining water and solubles which are not polymerized into fibrin. The bore holes can be distributed about the circumference and length of the rotating mould, e.g. every 5 to 20 mm of the length and two or more bore holes radially offset at one axial section of the mould. The bore holes can have diameters e.g. between 0.05 and 0.8 mm, e.g. between 0.1 and 0.6 or 0.5 or 0.4 mm. The rotating mould can e.g. have an inner diameter of 4 to 15 mm, e.g. 5 to 10 mm, preferably of 6 mm or 7 mm to 8 mm. Preferably, the outside surface of the mould is covered with a semipermeable membrane that retains fibrin but allows water to pass. The end sections of the mould preferably have a smaller inner diameter, e.g. projecting over the inner surface of the mould axially towards the longitudinal axis of the mould, for retaining solution on the inner surface of the rotating mould to a thickness corresponding to the end sections projecting over the inner surface of the rotating mould. The end sections can e.g. project over the inner surface of the rotating mould by 1.5 to 2.5 mm, e.g. for 2 mm, and optionally the end sections can have a central opening for introducing a fibrinogen containing composition and for allowing aqueous and dissolved compounds to leave the mould.

The sheath of fibrin that is produced by polymerizing a fibrinogen containing solution to fibrin in a rotating mould has a tensile strength of at least 12 to 50 kPa, e.g. 28 ± 10 kPa and has an elastic modulus of 2.8 ± 1.3 kPa. The tensile strength is measured along the longitudinal axis of the fibrin sheath, e.g. for a circumferentially closed fibrin sheath, and e.g. along the longitudinal axis of a strip forming a fibrin sheath by being wound around silk fibres.

It has been found that the sheath of compacted fibrin, herein also referred to as fibrin sheath, which is produced by the process, for growth of nerve cells preferably has pores between fibrin fibrils of 13 to 80 µm, e.g. of 35 ± 23 µm. These pore sizes have been found to favour the growth of nerve cells.

The silk fibers can terminate in the cross-section of the sheath, or the silk fibers can project out of the cross-section of the sheath, e.g. extend beyond the sheath. The ends of the silk fibers can be blunt ends, e.g. cut ends. Alternatively, the ends of the silk fibers can be loops, e.g. formed by a continuous loop between portions of silk fibers arranged inside and along the longitudinal axis of the sheath.

The implant of the invention has the advantage of being producible without containing cells, and accordingly the implant is devoid of cells, and can be stored in a dry state or in a hydrated state. Optionally, the implant is devoid of growth factors. Preferably, the implant is generated from autologous or allogenic plasma or whole blood, or from commercially available fibrinogen preparations, e.g. available as Haemocomplettan P from CSL Behring, Germany. Generally preferred, the fibrinogen, and the fibrin produced from the fibrinogen, are free from serum albumin. A preferred fibrinogen, and the fibrin produced from the fibrinogen, are produced by cryoprecipitation from blood plasma, as cryoprecipitation has been found to essentially avoid presence of serum albumin in fibrinogen preparations.

The fibrin that optionally fills the inner volume of the sheath and between the silk fibers is polymerized from a fibrinogen containing solution in the absence of pressure or g-force, but is allowed to polymerize under ambient pressure, e.g. under static conditions. This fibrin filling can have a tensile strength of 3.7 ± 1.7 kPa, and an elastic modulus of 0.06 ± 0.01 kPa, with interstices between fibrin fibrils of e.g. 140 to 220 µm, e.g. 160 ± 30 µm.

The elastic modulus is e.g. determined by measurement of the tensile strength as Δtensile force/ Δ length.

Subsequent to the production of the sheath by polymerizing fibrinogen from a solution in a rotating mould, the sheath can optionally be dried and, prior to or subsequent to arranging silk fibers in the sheath, be rehydrated. Drying of the sheath subsequent to producing it in the rotating mould is preferred, as it has been found that the drying increases the density of the fibrin sheath, and respectively reduces pores in the sheath, e.g. to pore sizes of 35 ± 23 µm.

Alternatively, silk fibers can be arranged in the sheath without prior drying of the sheath but essentially maintaining the water content of the sheath generated by the production of the sheath in the rotating mould.

Silk fibers can be arranged in the sheath after the sheath coming out of the mould has been dried, e.g. in a desiccator over dry NaCl or at 25% relative humidity at room temperature up to constant weight.

Herein, the silk fibers preferably are natural spider silk fibers or fibers spun from natural compounds only. Natural spider silk fibers are e.g. the major ampullate silk fiber, preferably obtained from spiders of the Nephila genus, e.g. Nephila clavipes. Silk fibers spun from natural compounds are silk fibers that are produced from natural compounds only, which are artificially spun, e.g. electro-spun, e.g. from dissolved silk protein or silk protein starting compounds, e.g. dissolved spider silk, dissolved silkworm silk, or dissolved silk obtained from insects, e.g. moths or butterflies, or mixtures of at least two of these as starting material. Therein, the dissolved silk protein can be protein having the amino acid sequence of a natural silk fiber, which protein is expressed in a micro-organism, e.g. expressed in bacteria, yeast or fungal cells, or cultivated mammalian cells, each genetically manipulated to express silk protein.

Herein, natural compounds include or consist of chemical compounds that are isolated from a natural source and chemical compounds that are obtained by chemical processes and are chemically identical to the compounds isolated from a natural source.

The invention is now described in greater detail by way of an example and with reference to the figures which schematically show in
- Fig. 1 an embodiment of the invention, and
- Fig. 2 a further embodiment of the invention.

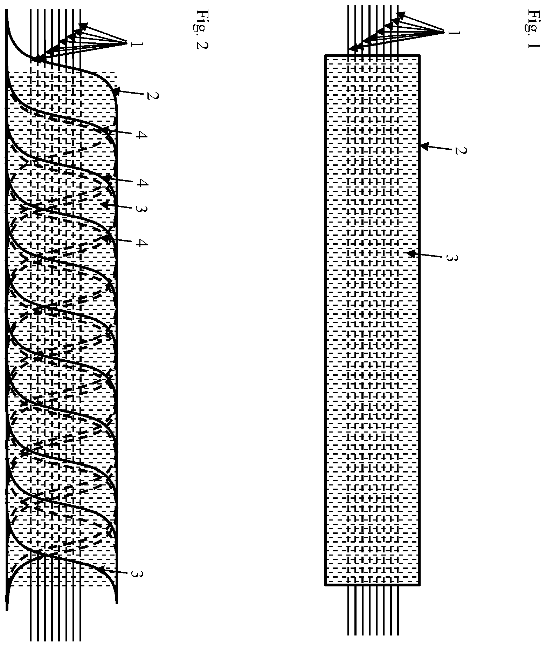

Fig. 1 shows an embodiment, in which silk fibers 1 are arranged inside a fibrin sheath 2, and the volume between the silk fibers 1 and the fibrin sheath 1 is filled by fibrin 3. As generally preferred, the fibrin sheath has a continuous wall, e.g. is tubular.

Fig. 2 shows an embodiment, in which the silk fibers 1 are arranged inside a fibrin sheath 2, and the volume between the silk fibers 1 and the fibrin sheath 2 is filled by fibrin 3, wherein the fibrin sheath 2 is made up of a strip-shaped fibrin that is wound around silk fibers. The strip-shaped fibrin can e.g. be prepared by cutting a tubular fibrin sheath 2 into one or more strips, e.g. into at least one spiral-shaped fibrin strip. Optionally, the strip-shaped fibrin that is wound around silk fibers is arranged such that the long edges 4 of the at least one strip overlap.

### Example: Production of the implant for bridging a gap in a nerve

A sheath consisting of fibrin was produced by introducing into a mould an aqueous mixture of fibrinogen solution, e.g. available as Haemocomplettan from CSL Behring, preferably a fibrinogen solution obtained by cryoprecipitation and separation from human plasma or whole blood, concurrently with thrombin as the activator for polymerization to fibrin. The mould was a 10 cm long metal tube, inner diameter 9 mm, with a total of 14 drainage bore holes, 0.5 mm diameter, distributed pairwise and opposite over the circumference, with a Teflon inset of two half-moulds forming a poly tetrafluoro ethylene coating of the inner mould surface to an inner diameter of 7 mm. The inner mould surface is cylindrical about a longitudinal axis. The end sections of the mould received inserts that reduced the inner diameter of end sections of the mould to 3 mm to prevent the fibrinogen solution from exiting the mould. The fibrinogen containing mixture was generated by concurrently introducing 5 mL aqueous fibrinogen solution at a flow rate of 120 mL/h having a concentration of 20 to 30 mg/mL fibrinogen, and an aqueous thrombin solution containing 40 mM CaCl₂, 1000 units aprotinin (obtained from Loxo), and 100 units thrombin (obtained from Baxter) at a flow rate of 80 mL/h, each to coupling leading to one common tubing that discharged into the mould while the mould was rotated at 5500 rpm, resulting in a centrifugal force about 120 x g. The common tube was connected to a 0.9 x 40 mm canula that was introduced axially in the mould and was retracted continuously from the mould during the discharge of the mixture of the fibrinogen solution and the thrombin solution. The mould was rotated about its longitudinal axis at about 10 000 rpm, resulting in a g-force of about 400 x g on the inner surface of the mould, for about 10 min. This process was preferably carried out at room temperature.

Subsequently, the polymerized sheath consisting of fibrin was removed from the mould. Prior to drying, i.e. directly after removal from the mould, the fibrin sheath had a maximum tensile strength of 10 to 17 kPa, e.g. 12 ± 2 kPa, and an elastic modulus of 0.8 to 1.5 kPa, e.g. 1.1 ± 0.3 kPa.

Subsequently, the fibrin sheath was dried over NaCl at room temperature. Preferably, prior to drying, an insert, e.g. of plastic or of steel, was inserted into the fibrin sheath to generate a dried sheath. The insert prevented a collapse of the fibrin sheath and it was found that the insert determined the inner diameter of the dried fibrin sheath. The dried sheath was re-hydrated in aqueous 40 mM CaCl₂, optionally containing 1 % vol/vol antibiotic solution (available as CellCultureGuard from PanReac Applichem, Germany), and containing 1000 units /mL aprotinin. This re-hydration solution is also preferred for storing the implant. Generally preferred, the water used in the process is aqua ad iniectabilia. Re-hydration was for 1 h at 4 °C, then the insert could be retracted, and the fibrin sheath could be stored in this solution at 4 °C. After the drying and re-hydration, the fibrin sheath had a maximum wall tension, also referred to as tensile strength, that was increased in comparison to a fibrin sheath directly after polymerization in the mould and without drying. In this example a maximum wall strength (tensile strength) of 12.4 to 46.1 kPa was determined for the dried and re-hydrated fibrin sheaths from several repetitions. The pores between fibrin fibrils of the dried and re-hydrated fibrin sheath from several repetitions were determined to have a diameter of 13-80 µm.

As the preferred example for silk fibers, natural spider silk, namely the major ampullate fiber of Nephila clavipes, was carefully inserted into a 4 mm long section of the fibrin sheath until 50 to 200 fibers were arranged longitudinally in the sheath. Subsequently, the sheath was filled with a mixture of 20 mg/mL fibrinogen in water, admixed with 10 units thrombin in 40 mM CaCl₂. This fibrin filling the inner volume of the fibrin sheath and enclosing the silk fibers within the sheath had a maximum tensile strength of 3.7 ± 1.7 kPa and an elastic modulus of 0.06 ± 0.01 kPa, with pores between fibrin fibres of 164 ± 31 µm.

This implant could be store in the storage solution containing aqueous 40 mM CaCl₂, optionally containing 1 % vol/vol antibiotics solution (available as CellCultureGuard), and containing 1000 units /mL aprotinin, at 4 °C without apparent deterioration of its properties.

The implant having a length of 3 to 4 mm was surgically inserted into a gap that was cut in the spinal cord of a rat, obeying animal care provisions for experimental animals. It was found that the implant resulted in the ingrowth of nerve cells, e.g. of dorsal root ganglia, and restoration of the nerve tissue.

In an in vitro assay, a section of the implant was incubated for 22 days in cultivated olfactory ensheathing cells (OEC) under static cell culture conditions. Analysis of the implants showed that the OEC migrate and proliferate on the spider silk fibers within the fibrin sheath. The proportion of living cells inside the implant was more than 98%, with no signs for toxicity detected.

As a further in vitro assay, live sections of rat spinal cord tissue were placed on top of implants, preferably on top of the cross-sectional end, horizontally arranged, of an implant and then cultivated under cell culture conditions. Analyses by immune fluorescence staining showed that neurons of the central nervous system as well as astrocytes, oligodendrocytes and microgliacells were found on silk fibers of the implant. It is assumed that these cells have migrated or grown out of the spinal cord tissue directly into the implant and along the silk fibers. Scanning electron microscopic pictures show that the nerve cells are oriented along the silk fibers.

## Claims

1. Implant for nerve regeneration, comprising a sheath consisting of fibrin, comprising silk fibers arranged in axial direction inside the sheath, wherein the inner volume of the sheath and between the silk fibers is filled with fibrin and wherein the sheath has tensile strength of at least 12 to 50 kPa.

2. Implant according to claim 1, **characterized in that** the sheath is circumferentially closed.

3. Implant according to claim 1, **characterized in that** the sheath is a strip that is wound around the silk fibers, wherein the long edges of the strip overlap.

4. Implant according to one of the preceding claims, **characterized in that** the sheath has pores between fibrin fibrils of 13 to 80 µm .

5. Implant according to one of the preceding claims, **characterized in that** the sheath consists of high density fibrin produced by polymerizing fibrinogen to fibrin in a rotationally symmetrical mould rotating about its longitudinal axis generating a g-force at its inner surface of at least 200 x g.

6. Implant according to one of the preceding claims, **characterized in that** the sheath is produced by polymerizing fibrinogen to fibrin, followed by drying the sheath before or after arranging silk fibers in the sheath to generate a dried sheath, and subsequently re-hydrating the sheath.

7. Implant according to one of the preceding claims, **characterized in that** the silk fibers are selected from spider silk fibers obtained from spiders, fibers spun from dissolved silk protein or silk protein starting compounds.

8. Implant according to one of the preceding claims, **characterized in that** the silk fibers project over the ends of the sheath and the silk fibers have cut ends or the silk fibers form a continuous loop between portions of silk fibers arranged inside and along the longitudinal axis of the sheath.

9. Implant according to one of the preceding claims, **characterized in that** it consists of the sheath, the silk fibers, and fibrin filling the sheath.

10. Implant according to one of the preceding claims, **characterized in that** the fibrin is produced from fibrinogen that is free from serum albumin, especially fibrinogen produced by cryoprecipitation from blood plasma.

11. Implant according to one of the preceding claims, **characterized in that** the fibrin filling the inner volume of the sheath and between the silk fibers has an elastic modulus of 80 to 120 Pa.

12. Process for producing an implant according to one of the preceding claims, comprising producing a sheath consisting of fibrin by polymerizing fibrinogen to fibrin in a mould rotating to generate a g-force of at least 100 x g at its inner surface, the sheath extending along a longitudinal axis, arranging silk fibers in the sheath to extend longitudinally along the sheath, and filling the inner volume of the sheath with fibrinogen and polymerizing the fibrinogen to fibrin.

13. Process according to claim 12, **characterized by** inserting an insert into the sheath and drying the sheath before or after arranging silk fibers in the sheath to generate a dried sheath, and, before or subsequent to arranging silk fibers in the sheath, re-hydrating the dried sheath to generate a re-hydrated sheath, optionally removing the insert from the sheath before or after re-hydrating.

14. Process according to one of claims 12 to 13, **characterized in that** the sheath is produced by polymerizing fibrinogen to fibrin, followed by drying the sheath before or after arranging silk fibers in the sheath to generate a dried sheath, before or after drying arranging silk fibers in the sheath, and subsequently re-hydrating the sheath and filling the inner volume of the sheath containing silk fibers with fibrinogen to produce fibrin filling the inner volume.

15. Process according to one of claims 12 to 14, **characterized in that** the sheath is circumferentially closed.

16. Process according to one of claims 12 to 14, **characterized in that** subsequent to polymerizing, the sheath is cut into a strip to generate a strip-shape sheath, and the silk fibers are arranged in the strip-shape sheath by winding the strip-shape sheath around longitudinally extending silk fibers, wherein the long edges of the strip-shape sheath overlap.

17. Process according to one of claims 12 to 16, **characterized in that** the implant comprising or consisting of the sheath, the silk fibers, and fibrin filling the sheath is hydrated and stored at above 0 °C and below 10 °C.

18. Process according to one of claims 12 to 17, **characterized in that** the fibrin is produced from fibrinogen that is free from serum albumin.

19. Process according to one of claims 12 to 18, **characterized in that** the fibrin is produced from fibrinogen produced by cryoprecipitation from blood plasma.

## Patentansprüche

1. Implantat zur Nervenregeneration, die eine aus Fibrin bestehende Hülle umfasst, umfassend Seidenfasern, die in axialer Richtung innerhalb der Hülle angeordnet sind, wobei das Innenvolumen der Hülle und zwischen den Seidenfasern mit Fibrin gefüllt ist und wobei die Hülle eine Zugfestigkeit von mindestens 12 bis 50 kPa aufweist.

2. Implantat nach Anspruch 1, **dadurch gekennzeichnet, dass** die Hülle in umfänglich geschlossen ist.

3. Implantat nach Anspruch 1, **dadurch gekennzeichnet, dass** die Hülle ein Streifen ist, der um die Seidenfasern gewickelt ist, wobei sich die langen Kanten des Streifens überlappen.

4. Implantat nach einem der vorhergehenden Ansprüche, **dadurch gekennzeichnet, dass** die Hülle Poren zwischen Fibrinfibrillen von 13 bis 80 µm aufweist.

5. Implantat nach einem der vorhergehenden Ansprüche, **dadurch gekennzeichnet, dass** die Hülle aus hochdichtem Fibrin besteht, das durch Polymerisation von Fibrinogen zu Fibrin in einer rotationssymmetrischen Form hergestellt wird, die sich um ihre Längsachse dreht und an ihrer Innenfläche eine g-Kraft von mindestens 200 x g erzeugt.

6. Implantat nach einem der vorhergehenden Ansprüche, **dadurch gekennzeichnet, dass** die Hülle durch Polymerisation von Fibrinogen zu Fibrin hergestellt ist, gefolgt von Trocknen der Hülle vor oder nach dem Anordnen von Seidenfasern in der Hülle, um eine getrocknete Hülle zu erzeugen, und anschließendem Rehydrieren der Hülle.

7. Implantat nach einem der vorhergehenden Ansprüche, **dadurch gekennzeichnet, dass** die Seidenfasern ausgewählt sind aus Spinnenseidenfasern, die aus Spinnen gewonnen werden, Fasern, die aus gelöstem Seidenprotein gesponnen werden, oder Seidenprotein-Ausgangsverbindungen.

8. Implantat nach einem der vorhergehenden Ansprüche, **dadurch gekennzeichnet, dass** die Seidenfasern über die Enden der Hülle hinausragen und die Seidenfasern abgeschnittene Enden haben oder die Seidenfasern eine durchgehende Schlaufe zwischen Abschnitten von Seidenfasern bilden, die innerhalb und entlang der Längsachse der Hülle angeordnet sind.

9. Implantat nach einem der vorhergehenden Ansprüche, **dadurch gekennzeichnet, dass** es aus der Hülle, den Seidenfasern und dem Fibrin, das die Hülle ausfüllt, besteht.

10. Implantat nach einem der vorhergehenden Ansprüche, **dadurch gekennzeichnet, dass** das Fibrin aus serumalbuminfreiem Fibrinogen, insbesondere aus durch Kryopräzipitation aus Blutplasma gewonnenem Fibrinogen, hergestellt ist.

11. Implantat nach einem der vorhergehenden Ansprüche, **dadurch gekennzeichnet, dass** das Fibrin, das das Innenvolumen der Hülle und zwischen den Seidenfasern ausfüllt, einen Elastizitätsmodul von 80 bis 120 Pa aufweist

12. Verfahren zur Herstellung eines Implantats nach einem der vorhergehenden Ansprüche, umfassend die Herstellung einer aus Fibrin bestehenden Hülle durch Polymerisation von Fibrinogen zu Fibrin in einer Form, die rotiert, um eine g-Kraft von mindestens 100 x g an ihrer inneren Oberfläche zu erzeugen, wobei sich die Hülle entlang einer Längsachse erstreckt, Anordnen von Seidenfasern in der Hülle, so dass sie sich in Längsrichtung entlang der Hülle erstrecken, und Füllen des Innenvolumens der Hülle mit Fibrinogen und Polymerisieren des Fibrinogens zu Fibrin.

13. Verfahren nach Anspruch 12, **gekennzeichnet durch** Einsetzen eines Einsatzes in die Hülle und Trocknen der Hülle vor oder nach dem Anordnen von Seidenfasern in der Hülle, um eine getrocknete Hülle zu erzeugen, und Rehydrieren der getrockneten Hülle vor oder nach dem Anordnen von Seidenfasern in der Hülle, um eine rehydrierte Hülle zu erzeugen, gegebenenfalls Entfernen des Einsatzes aus der Hülle vor oder nach dem Rehydrieren.

14. Verfahren nach einem der Ansprüche 12 bis 13, **dadurch gekennzeichnet, dass** die Hülle durch Polymerisieren von Fibrinogen zu Fibrin hergestellt wird, gefolgt von Trocknen der Hülle vor oder nach dem Anordnen von Seidenfasern in der Hülle, um eine getrocknete Hülle zu erzeugen, vor oder nach dem Trocknen Anordnen von Seidenfasern in der Hülle, und anschließendem Rehydrieren der Hülle und Füllen des Innenvolumens der Hülle, die Seidenfasern enthält, mit Fibrinogen, um Fibrin zu erzeugen, das das Innenvolumen ausfüllt.

15. Verfahren nach einem der Ansprüche 12 bis 14, **dadurch gekennzeichnet, dass** die Hülle umfänglich geschlossen ist.

16. Verfahren nach einem der Ansprüche 12 bis 14, **dadurch gekennzeichnet, dass** nach dem Polymerisieren die Hülle in einen Streifen geschnitten wird, um eine streifenförmige Hülle zu erzeugen, und die Seidenfasern in der streifenförmigen Hülle angeordnet werden, indem die streifenförmige Hülle um sich in Längsrichtung erstreckende Seidenfasern gewickelt wird, wobei sich die langen Kanten der streifenförmigen Hülle überlappen.

17. Verfahren nach einem der Ansprüche 12 bis 16, **dadurch gekennzeichnet, dass** das Implantat, das die Hülle, die Seidenfasern und das die Hülle ausfüllende Fibrin umfasst oder daraus besteht, hydratisiert und bei über 0 °C und unter 10 °C gelagert wird.

18. Verfahren nach einem der Ansprüche 12 bis 17, **dadurch gekennzeichnet, dass** das Fibrin aus Fibrinogen hergestellt wird, das frei von Serumalbumin ist.

19. Verfahren nach einem der Ansprüche 12 bis 18, **dadurch gekennzeichnet, dass** das Fibrin aus Fibrinogen hergestellt wird, das durch Kryopräzipitation aus Blutplasma gewonnen ist.

## Revendications

1. Implant pour la régénération des nerfs, comprenant une gaine constituée de fibrine, comprenant des fibres de soie disposées dans le sens axial à l'intérieur de la gaine, dans lequel le volume intérieur de la gaine et entre les fibres de soie est rempli de fibrine et dans lequel la gaine a une résistance à la traction d'au moins 12 à 50 kPa.

2. Implant selon la revendication 1, **caractérisé par le fait que** la gaine est fermée de manière circonférentielle.

3. Implant selon la revendication 1, **caractérisé par le fait que** la gaine est une bande enroulée autour des fibres de soie, dans laquelle les bords longs de la bande se chevauchent.

4. Implant selon l'une des revendications précédentes, **caractérisé par le fait que** la gaine présente des pores entre les fibrilles de fibrine de 13 à 80 µm .

5. Implant selon l'une des revendications précédentes, **caractérisé par le fait que** la gaine est constituée de fibrine de haute densité produite par polymérisation du fibrinogène en fibrine dans un moule à symétrie de révolution tournant autour de son axe longitudinal et générant une force g sur sa surface interne d'au moins 200 x g.

6. Implant selon l'une des revendications précédentes, **caractérisé par le fait que** la gaine est produite par polymérisation du fibrinogène en fibrine, suivie d'un séchage de la gaine avant ou après avoir disposé des fibres de soie dans la gaine pour générer une gaine séchée, puis d'une réhydratation de la gaine.

7. Implant selon l'une des revendications précédentes, **caractérisé par le fait que** les fibres de soie sont choisies parmi les fibres de soie d'araignée obtenues à partir d'araignées, les fibres filées à partir de protéines de soie dissoutes ou de composés de départ de protéines de soie.

8. Implant selon l'une des revendications précédentes, **caractérisé par le fait que** les fibres de soie font saillie sur les extrémités de la gaine et que les fibres de soie ont des extrémités coupées ou que les fibres de soie forment une boucle continue entre des portions de fibres de soie disposées à l'intérieur et le long de l'axe longitudinal de la gaine.

9. Implant selon l'une des revendications précédentes, **caractérisé en ce qu'**il est constitué de la gaine, des fibres de soie et de la fibrine remplissant la gaine.

10. Implant selon l'une des revendications précédentes, **caractérisé par le fait que** la fibrine est produite à partir de fibrinogène exempt d'albumine sérique, notamment de fibrinogène produit par cryoprécipitation à partir de plasma sanguin.

11. Implant selon l'une des revendications précédentes, **caractérisé par le fait que** la fibrine remplissant le volume intérieur de la gaine et entre les fibres de soie a un module d'élasticité de 80 à 120 Pa

12. Procédé de fabrication d'un implant selon l'une des revendications précédentes, comprenant la production d'une gaine constituée de fibrine par polymérisation du fibrinogène en fibrine dans un moule tournant pour générer une force g d'au moins 100 x g sur sa surface intérieure, la gaine s'étendant le long d'un axe longitudinal, la disposition des fibres de soie dans la gaine pour s'étendre longitudinalement le long de la gaine, et le remplissage du volume intérieur de la gaine avec du fibrinogène et la polymérisation du fibrinogène en fibrine.

13. Procédé selon la revendication 12, **caractérisé par** l'insertion d'un insert dans la gaine et le séchage de la gaine avant ou après la disposition des fibres de soie dans la gaine pour générer une gaine séchée, et, avant ou après la disposition des fibres de soie dans la gaine, la réhydratation de la gaine séchée pour générer une gaine réhydratée, en retirant éventuellement l'insert de la gaine avant ou après la réhydratation.

14. Procédé selon l'une des revendications 12 à 13, **caractérisé par le fait que** la gaine est produite par polymérisation du fibrinogène en fibrine, suivi du séchage de la gaine avant ou après avoir disposé les fibres de soie dans la gaine pour générer une gaine séchée, avant ou après le séchage avoir disposé les fibres de soie dans la gaine, puis réhydratation de la gaine et remplissage du volume intérieur de la gaine contenant les fibres de soie avec du fibrinogène pour produire de la fibrine remplissant le volume intérieur.

15. Procédé selon l'une des revendications 12 à 14, **caractérisé par le fait que** la gaine est fermée circonférentiellement.

16. Procédé selon l'une des revendications 12 à 14, **caractérisé par le fait qu'**après polymérisation, la gaine est découpée en bande pour générer une gaine en forme de bande, et les fibres de soie sont disposées dans la gaine en forme de bande par enroulement de la gaine en forme de bande autour de fibres de soie s'étendant longitudinalement, dans laquelle les bords longs de la gaine en forme de bande se chevauchent.

17. Procédé selon l'une des revendications 12 à 16, **caractérisé par le fait que** l'implant comprenant ou constitué de la gaine, des fibres de soie et de la fibrine remplissant la gaine est hydraté et stocké à une température supérieure à 0 °C et inférieure à 10 °C.

18. Procédé selon l'une des revendications 12 à 17, **caractérisé par le fait que** la fibrine est produite à partir de fibrinogène exempt d'albumine sérique.

19. Procédé selon l'une des revendications 12 à 18, **caractérisé par le fait que** la fibrine est produite à partir du fibrinogène produit par cryoprécipitation à partir du plasma sanguin.
